Europäisches Patentamt

European Patent Office

Office européen des brevets

(11) Publication number: **0 147 198**
**B1**

(12) **EUROPEAN PATENT SPECIFICATION**

(45) Date of publication of patent specification: 07.11.90

(21) Application number: 84308981.4

(22) Date of filing: 20.12.84

(51) Int. Cl.⁵: **C 12 N 15/81,** C 12 C 11/00, C 12 P 7/06, C 12 P 21/02 // C12N9/24, C12N9/86

(54) Fermentation processes and their products.

(30) Priority: 22.12.83 GB 8334261

(43) Date of publication of application:
03.07.85 Bulletin 85/27

(45) Publication of the grant of the patent:
07.11.90 Bulletin 90/45

(84) Designated Contracting States:
AT BE CH DE FR GB IT LI LU NL SE

(56) References cited:
EP-A-0 096 491
GB-A-2 094 341

BREWERS' GUARDIAN, September 1984, pages 34-37; R.S. TUBB: "Genetic development of yeast strains"

CHEMICAL ABSTRACTS, vol. 98, no. 7, February 1983, page 179, abstract no. 47922r, Columbus, Ohio, US; R.A. IRVING et al.: "Development of an amylolytic Saccharomyces cerevisiae by genetic engineering", & PROC.-BIOENERGY R & D SEMIN 1982, 4th, 541-5

(73) Proprietor: Delta Biotechnology Limited
137 High Street
Burton on Trent DE14 1JZ (GB)

(72) Inventor: Molzahn, Stuart, William
91 The Plain
Brailsford Derbyshire DE6 3BR (GB)

(74) Representative: Bassett, Richard Simon et al
ERIC POTTER & CLARKSON St. Mary's Court St. Mary's Gate
Nottingham NG1 1LE (GB)

(56) References cited:

AGRICULTURAL AND BIOLOGICAL CHEMISTRY, vol. 47, no. 11, November 1983, pages 2689-2692; I. YAMASHITA et al.: "Molecular cloning of a glucoamylase-producing gene in the yeast Saccharomyces"

CURRENT GENETICS, vol. 2, November 1980, pages 109-113; J.J. PANTHIER et al.: "Cloned beta-galactosidase gene of Escherichia coli is expressed in the yeast Saccharomyces cerevisiae"

# EP 0 147 198 B1

(56) References cited:

CHEMICAL ABSTRACTS, vol. 99, no. 19, 7th
November 1983, page 164, abstract no. 153176f,
Columbus, Ohio, US; B.A. CANTWELL et al.:
"Molecular cloning and expression of a Bacillus
subtilis beta-glucanase gene in Escherichia coli",
& GENE 1983, 23(2), 211-19

CHEMICAL ABSTRACTS, vol. 101, no. 23,
December 1984, page 153, abstract no. 205283v,
Columbus, Ohio, US; E. HINCHLIFFE et al.:
"Expression of the cloned endo-1,3-1,4-beta-
glucanase gene of Bacillus subtilis in
Saccharomyces cerevisiae", & CURR. GENET.
1984, 8(6), 471-5

ABSTRACTS OF THE ANNUAL MEETING OF
THE AMERICAN SOCIETY FOR MICROBIOLOGY
(USA), vol. 81, 1981, page 116, ref. H19; S.K.
PICATAGGIO et al.: "The cloning of trichoderma
reesei genomic DNA in Escherichia coli HB101"

## Description

This invention relates to fermentation processes and their products, and more particularly to the production of alcohol, i.e. ethanol, by fermentation of sugars with yeast.

In the manufacture of alcohol by fermentation, sugars in aqueous solution are converted into ethanol by fermentation with yeast. The yeast grows during the fermentation and although a small proportion of the yeast may be used in a subsequent fermentation process, the remainder of the yeast constitutes an excess that must be disposed of. While this excess yeast has some uses e.g. in animal feedstufs and the manufacture of yeast extracts, the quantity of excess yeast produced is large and its market value is relatively low.

Large scale fermentations of this kind fall into three broad categories:

(1) Fermentations in which the fermented aqueous medium obtained is the desired end product. Into this category fall ordinary brewing processes for the production of beer (a term which, as used herein, includes ales, stouts, lagers and other fermented drinks based on malt), cider and other fermented drinks.

(2) Fermentations in which the desired end product is a distilled drinkable alcoholic concentrate. Into this category fall fermentations for production of whiskies, brandies and other spirits, and alcohol for use in fortifying other drinks.

(3) Fermentation for the production of alcohol for industrial use. Into this category falls fermentations carried out in some countries on a large scale for the production of fuel alcohol. The production of excess yeast is a characteristic of all these industrial processes.

Considerable interest has been shown in recent years in the genetic modification of microorganisms so that they become able to produce heterologous proteins and peptides, that is to say proteins and peptides which are not produced by their natural genetic constituents. A variety of microorganisms have been used for such genetic manipulation, and, amongst these, yeasts have attracted a certain amount of interest. However, yeasts used in laboratory experiments are not normally the same as the yeasts used in large scale industrial fermentations involving the production of alcohol, and the conditions of growth of yeast in the laboratory are very different from those encountered by yeasts in an industrial alcoholic fermentation.

The present invention is based on the discovery that it is possible to use, in an industrial fermentation involving the production of alcohol, genetically modified yeast capable of expressing a heterologous protein or peptide. Surprisingly, it has been found that the use of such yeast is compatible with industrial fermentation conditions. This means that the excess yeast obtained in the fermentation provides a source of the heterologous protein or peptide and thus has much enhanced industrial value. Further, since the alcohol product remains the principle objective of the fermentation, and the conventional equipment can largely be used with little alteration, the additional cost of producing the higher value yeast product is small, so that the new process may provide an economically viable route to heterologous proteins or peptides which, although valuable, do not command a premium price.

The present invention accordingly provides a process for the production of ethanol and a protein or peptide which is heterologous to yeast which comprises fermenting an aqueous sugar-containing medium with an industrial *S. cerevisiae* or *S. carlbergensis* yeast strain with has been genetically modified to be capable of expressing a heterologous protein or peptide, recovering the ethanol so formed, and obtaining the said heterologous protein or peptide from the fermentation products.

The yeasts are a group of lower eukaryotic micro-organisms showing biological and biochemical diversity. In common usage the term "yeast" is used to describe strains of *Saccharomyces cerevisiae* that have commercial value in baking, brewing and distilling. Related yeasts are used in wine making and sake brewing, as well as in the production of fuel alcohol from sucrose or hydrolysed starch.

All the yeasts used for brewing, baking and distilling may be taxonomically classified as *Saccharomyces cerevisiae*. Included within this classification are the top fermenting ale yeasts (*S. cerevisiae*) and the bottom-fermenting lager yeasts (*S. uvarum* or *S. carlsbergensis*).

In a strict sense brewers yeast is differentiated from all other yeasts in that it is a yeast strain which is used to make beer, i.e. a strain of yeast used currently in a beer manufacturing process. Such yeasts must be able to produce a palatable acceptable beer by their fermentative action upon hopped malt extract (brewers wort). The primary products of this fermentation are ethanol and carbon dioxide, which are essential constituents of beer. However, not all yeasts belonging to the species *S. cerevisiae* are capable of fulfilling these requirements. Indeed, the critical factor in this respect is believed to be the ability of the yeast strain to form in subtly balanced proportions, quantitatively miner metabolic products such as esters, acids, higher alcohols and ketones. A yeast may be unsuitable for brewing because one or more or these minor metabolic products is produced in excessive amounts, either in absolute terms or relative to one another. (Rainbow, C.A., 1970, In "The Yeasts", eds, Rose, A.H. & Harrison J.S. Vol. 3, p. 147).

In a general sense brewers yeast is differentiated from other yeasts by the properties which it possesses. Most strains of industrial yeast, unlike laboratory yeast, are incapable of undergoing mating; they are said to be homothallic. Industrial yeasts are usually aneuploid or polyploid, and there is therefore a reduced incidence at which gene mutations are phenotypically detected.

Most polyploid strains do not sporulate or they produce spores of very low viability, thus frustrating any meaningful genetic analysis. These factors together tend to confer a measure of phenotypic stability on industrial yeasts which may contribute to their selection for industrial application. Similarly gene dosage which is associated with high ploidy may contribute to the general fitness of such strains for fermentation as compared to haploids and diploids, which generally ferment poorly.

In addition, brewers yeasts have certain technological behaviour which equips them well for interacting with their normal environment, brewers' hopped wort.

The manner in which the new process is operated depends on the type of industrial fermentation. Where the fermentation is designed to produce an aqueous potable liquid such as beer, at the end of the fermentation, the fermented liquid is separated from the yeast (and normally any other solid material present in the fermented medium). In these circumstances, it is clearly desirable, and indeed normally essential, that the heterologous protein or peptide shall not become dissolved in the fermented liquid, since it is normally unacceptable for the heterologous protein or peptide to be present in a liquid which is to be drunk. In such circumstances, the heterologous protein or peptide may be obtained from the yeast cells. Where, however, the alcohol is recovered by distillation, as is the case in the second and third types of industrial fermentation mentioned above, it may be acceptable, and even desirable, for the protein or peptide to be present in the fermented liquid in dissolved form.

The yeast strain used in the new process must, of course, be suitable for the type of industrial fermentation contemplated. This objective may be secured by carrying out the genetic modification on a yeast strain which is known to have the desired characteristics, since it has been found that the desirable characteristics which make a yeast strain suitable for a particular type of industrial fermentation are not normally lost during the genetic modification. For example, where the fermentation is one for producing beer, the yeast strain chosen for genetic modification is preferably a known strain of brewers' yeast currently used in such fermentations. As already noted, such industrial strains of brewers yeast have characteristics different from those of "laboratory yeast", including in particular the ability to ferment hopped brewers wort.

Brewers wort is essentially a hot water extract of malted barley or other grains prepared by steeping and germination and flavoured with hops. The most important parametes with respect to yeast growth and metabolism are carbohydrate and nitrogen (and amino acid) composition. These vary from country to country and brewery to brewery, see. e.g.. "Malting and Brewing Science", Vol. 2, Hopped Wort and Beer; by Hough, J.S., Briggs, D.E., Steven R. and Young, T.W., 1982, Chapman and Hall, London and New York, p. 456—498. In general it may be said that brewers wort contains 5 to 10 g of total fermentable sugars per 100 ml of wort, at least half of which is maltose.

Additional factors which influence yeast growth and performance are: (1) *Growth factors*. These include substances like biotin, thiamine, riboflavin, pyridoxine, pantothenic acid and nicotinic acid. In general brewers wort is a rich source of these factors, which are depleted during yeast growth. (2) *Minerals*. The mineral requirements of brewers yeast resemble those of most living organisms. Brewers wort meets these requirements, supplying trace amounts of metal ions such as iron, potassium, magnesium, zinc, manganese and copper, which are essential for vital metabolic enzymes.

The most significant difference between a laboratory culture medium and a brewers wort is the sugar composition of the medium. Most laboratory media utilise glucose as the chief source of carbohydrate, whereas maltose is the chief fermentable constituent of wort.

Brewery fermentations normally take the form of anaerobic (oxygen free) fermentations. However, oxygen is a prime requirement for yeast growth in the initial stages of fermentation. Most laboratory fermentations are designed to maximise the yeast biomass yield, whereas beer fermentations concentrate upon ethanol yield and product flavour. Thus the inoculation rate ("pitching rate") of a beer fermentation is higher than would normally be used in the laboratory. Consequently, the number of cell doublings (cell generations) is reduced to between 2 and 4 per fermentation.

The fermentation of beer wort is normally carried out at a temperature within the range of 8 to 25°C, a temperature at the upper end of this range, e.g. 15 to 25°C being used when the product is ale, and a temperature of e.g. 8 to 15°C being used where the product is larger. Under laboratory conditions, yeasts are cultivated at significantly higher temperature, e.g. 25 to 35°C.

Similarly, where the industrial fermentation is one for the production of alcohol which is separated by distillation, it is necessary to use genetically modified yeast obtained from a strain suitable for such fermentation. In such fementations, the source of sugars may be, for example, grain, potatoes, cassava, sugar cane, or sugar beet and may optionally have been pre-treated, e.g. by chemical or enzymic hydrolysis, to convert cellulose and/or starch therein into fermentable sugars.

The genetic modification of yeast may be effected in a known manner. Suitable methods are discribed in the literature, and particular methods are given in the Examples below.

A wide range of heterologous proteins or peptides may be chosen for expression in the yeast. By way of example mention may be made of enzymes such as beta-lactamase, beta-glucanase, and beta-galactosidase. Other useful heterologous proteins and peptides include materials of human origin and/or useful in therapy, such as human serum albumin and immunoglobulins. Methods are described in the literature for the genetic modifications of microorganisms to enable them to express such proteins and peptides.

The heterologous protein or peptide made available by the genetically modified yeast may be used in several different ways. In the simplest case, the protein or peptide is retained by the yeast in the yeast cells and the latter are used as such. Normally, however, it is preferred to isolate the heterologous protein or peptide. Where the latter is excreted by the yeast into the surrounding medium, the fermented medium is worked up for isolation of protein or peptide. As already noted, this method is normally unsuitable where the fermented medium is to be consumed, e.g. as a beverage. In such a case, the desired protein or peptide is obtained from the yeast produced during the fermentation. For example, the yeast cells may be ruptured to release their contents, and the protein or peptide then isolated from the latter.

The following examples illustrate the invention in more detail. The accompanying drawings shows gene maps illustrating the formation of a plasmid used in one example. These examples describe the modification of brewers yeast so that it produces the heterologous proteins beta-lactamase and/or beta-glucanase and the use of the modified yeast in a brewing process.

ß-lactamase is the name given to a group of proteins that constitute enzymes operative to catalyse the hydrolysis of the amide bond in the ß-lactam ring of 6-amino-penicillanic acid or 7-amino-cephalosporanic acid and of their N-acyl derivatives. Such derivatives are penicillins and cephalosporins, generally known as ß-lactam antibiotics (Citri, N., 1971, "The Enzymes", 3rd edition, ed. Boyer, P.A., *IV*, p 23).

β-lactamase is widespread amongst the various bacterial species, being found in both Gram-negative and Gram-positive bacteria. The gene specifying the production of β-lactamase has been variously assigned to both chromosomal and extrachromosomal elements. In enteric bacteria a β-lactmase gene can frequently be acquired by infection with an extrachromosomal particle in the form of a plasmid and constiting a resistance factor (or R-factor). One such R-factor carrying a β-lactamase gene, and thus conferring resistance upon its host bacterium to β-lactam antibiotics, is R1 (Meynell, E. & Datta, N., 1966, *Genetical Research*, 7, p 134). This plasmid was identified in a clinical isolate of *Salmonella paratyphi* B (Meynell, E. & Datta, N., 1966, *Genetical Research*, 7, p 134). The species specificity of β-lactamase has been brought into question since R-factors are capable of mediating their own transfer, and thus the transfer of the β-lactamase gene among the Enterobacteriaceae (enteric bacteria) (Datta, N. & Richmond, M.H., 1966, *Biochemical Journal*, 98, p 204).

With the advant of genetic engineering (recombinant DNA technology) there has developed a requirement for easily manipulated plasmid vectors for use in DNA cloning. The β-lactamase gene present on plasmid R1 has been introduced into new plasmids in the construction of novel cloning vectors. One such vector is RSF 2124 (So, M. *et al.*, 1975, *Molecular and General Genetics, 142*, p 239) constructed from the plasmid Col E1 and a derivative of R1, R1 *drd 19* (Meynell, E. & Datta, N., 1967, *Nature, 214*, p 885).

RSF 2124 has been manipulated subsequently to produce the plasmid vector pBR322 (Bolivar, F. *et al*, 1977, *Gene, 2*, p 95), which has been further manipulated to form pAT153 (Twigg, A.A. & Sherratt, D., 1980, *Nature, 283*, p 216). All these plasmid vectors retain the β-lactamase gene of R1 and are capable of specifying the production of β-lactamase enzyme in *Escherichia coli*.

Additional manipulation of plasmid cloning vectors derived from pBR322, and therefore possessing the β-lactamase gene of R1, has been necessary to construct plasmids capable of transforming yeast (i.e. of being introduced into yeast). Thus, for example, the plasmid pAT153 (Twigg, A.J. & Sherratt, D., 1980, *Nature, 283*, p 216) has been attached to segments of yeast chromosomal DNA (*LEU-2* gene of *Saccharomyces cerevisiae* specifying the production of β-iso-propyl-malate-dehydrogenase, an enzyme involved in the biosynthesis of leucine) and 2 μm plasmid DNA (2μm is an endogenous plasmid of yeast) to form plasmid pJDB207 (Beggs, J.D., 1981, "Molecular Genetics in Yeast", eds. von Wettstein, D., Stenderup, A., Kielland-Brandt, M. & Friis, J., *Alfred Benzon Symposium No. 16*, Munksgaard, Copenhagen, p 383).

β-lactamase was the first heterologous protein to be expressed in *S. cerevisiae* (Hollenberg, C.P., 1979, *ICN-UCLA Symposium Molecular and Cellular Biology, 15*, p 325; Hollenberg, C.P., 1979, "Plasmids of Medical, Environmental and Commercial Importance", eds, Timmis, K.N. & Puhler, A., Elsevier, p 481). The bacterial ampicillin-resistance gene specifying the production of β-lactamase enzyme originated from plasmid pBR325, a derivative of pBR322, and therefore ultimately from *Salmonella paratyphi* B (see earlier references). The β-lactamase protein synthesised in *S. cerevisiae* has been purified 100-fold over crude extracts, and its enzymic activity, molecular weight and binding to specific antibodies have been shown to be indistinguishable from the purified protein from *E. coli* (Roggenkamp, R. *et al*, 1981, *PNAS USA, 78*, p 4466). The level of β-lactamase expression in yeast is low due to the weak function of the bacterial gene promoter (control region of the gene); however, gene expression can be greatly enhanced by the use of the ADH1 promoter (alcohol dehydrogenase) of yeast (Hollenberg, C.P. *et al*, 1983, "Gene Expression in Yeast", eds. Korhola, M. & Vaisanen, E., *Proceedings of the Alko Yeast Symposium*, Helsinki, p 73).

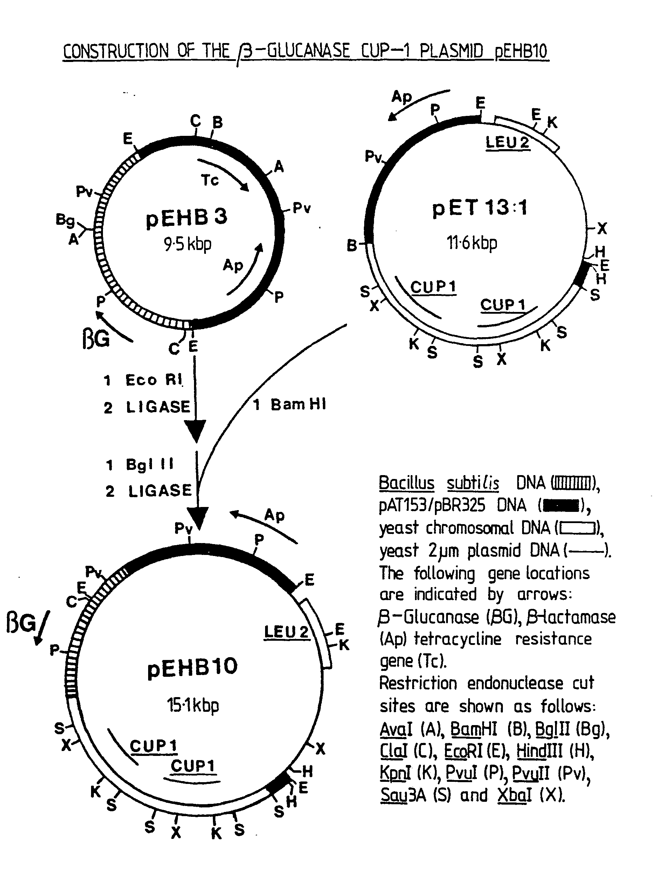

Yeast transformation (that is the introduction of DNA into yeast) can be a relatively inefficient process, with success depending upon a suitable selection system. Most plasmids, currently in use for yeast transformation are selectable, because they carry a wild-type gene which complements an auxotrophic mutation in the chosen recipient strain which has been a laboratory haploid strain of *S. cerevisiae*. However, brewers' yeasts are prototrophic and have no auxotrophic requirements. To select transformants in brewers' yeast it is necessary to have a dominant gene conferring the ability to grow in otherwise adverse conditions. *CUP-1* is a dominant yeast gene, specifying the production of a protein capable of chelating copper ions. This gene has been cloned on the yeast/*E. coli* shuttle vector pJDB207, by insertion of restriction-endonuclease-*Sau*3A-generated DNA fragments from strain X2180-1A to form plasmid pET13:1 (Henderson, R.C.A., 1983, "The Genetics and Applications of Copper Resistance in Yeast", Ph.D. thesis, University of Oxford). A genetic map of pET13:1 is included in the accompanying drawing. Plasmid pET13:1 carries the *LEU-2* and *CUP-1* chromosomal genes of yeast and the 2μm yeast plasmid origin of DNA replication as well as DNA derived from plasmid pAT153; consequently pET13:1 harbours the bacterial β-lactamase gene which is known to express β-lactamase in yeast. Henderson (1983) describes in some detail methods for transforming brewers' yeast (ale yeast and lager yeast) with plasmid pET13:1. He also described the screening of brewers' yeast transformants for β-lactamase activity using a starch iodide plate assay described below. Clearly the bacterial β-lactamase protein is produced in brewer's yeast transformed with pET13:1 and can be detected when transformants are grown upon the appropriate indicator medium.

The genetic modification of a particular strain of brewers' yeast, by the introduction into it of the plasmid pET13:1, will now be described. The yeast used was NCYC 240, which is an ale yeast which is available to the public from the National Collection of Yeast Cultures (Agricultural Research Council, Food Research Institute, Colney Lane, Norwich, England).

Before strain NCYC 240 could be transformed with plasmid pET13:1 (CUP-1/β-lactamase) its sensitivity to copper was assessed. To this end, samples of NCYC 240 were patched on YED glucose or YED glucose agar (1% w/v yeast extract, 2% w/v peptone, 2% w/v glucose, solidified with 2% w/v agar) and grown for 2 days at 28°C. They were then replica plated to NEP agar medium ($MgSO_4.7H_2O$ 2 g/l, $(NH_4)_2SO_4$ 2 g/l, $KH_2PO_4$ 3 g/l, $CaCl_2.2H_2O$ 0.25 g/l, yeast extract 2 g/l, peptone 3 g/l, glucose 40 g/l solidified with 2% agar. Naiki, N. & Yamagata, S., 1976, *Plant and Cell Physiology, 17*, p 1281) containing increasing concentrations of copper sulphate ($CuSO_4$). The strain tested did not grow on NEP containing 0.1 mM $CuSO_4$. It was therefore concluded that in excess of 0.1 mM $CuSO_4$ in NEP would be sufficient to select for copper resistant transformants of brewers' yeast.

Plasmid DNA of pET13:1 was isolated from the bacterium *Escherichia coli* K-12 strain JA221 (*recA1, leuB6, trp E5, hsdR−, hsDM+, lacY*. Beggs, J.D., 1978, *Nature, 275*, p 104) by caesium chloride/ethidium bromide equilibrium gradient centrifugation of cleared cell lysates using the method of Clewell, D.B. & Helinski, D.R. (1967, *Proceedings of the National Academy of Science, USA, 62*, p 1159) with the modifications of Zahn, G. *et al.* (1977, *Molecular and General Genetics, 153*, p 45).

Samples of NCYC 240 were prepared for transformation with pET13:1 by each of two methods: (A) the method of Beggs, J.D. (1978, *Nature, 275*, p 104), and (B) the method described by Henderson R.C.A. (1983, "The Genetics and Applications of Copper Resistance in Yeast", Ph.D. thesis, University of Oxford) with the exception that the protoplasting enzyme used was Zymolyase (40 μg/ml) Kirin Brewery Co. Ltd.). 100 μl of yeast spheroplasts produced by methods A and B were mixed with 15 μl of pET13:1 DNA (approximately 250 μg DNA/ml) and treated with polyethylene glycol (1 ml 40% PEG 4000 in 10 mM $CaCl_2$, 10 mM Tris/HCl pH 7—6). After the treatment with polyethylene glycol, cells were spun down and gently resuspended in 500 μl NEP glucose medium containing 1.2 M sorbitol. Following incubation for one hour at 28°C, cells were added to 10 ml of molten NEP glucose 3% agar containing 0.3 mM $CuSO_4$ and 1.2 sorbitol. This was then poured onto NEP glucose 2% agar medium containing 1.2 M sorbitol and 0.3 mM $CuSO_4$. Transformation plates were incubated for four to five days at 28°C after which time yeast colonies arising on the selective copper medium were picked off and patched upon NEP glucose agar containing 0.3 mM $CuSO_4$. These patched colonies were designated putative pET13:1 transformants, and were checked as described below to confirm that they were genuine brewers' yeast transformants. The frequencies of transformation for each of the two methods A and B for NCYC 240 were <4 transformants/μg DNA and 20 transformants/μg DNA respectively.

It is usual when attempting to confirm that a strain of yeast or bacteria is a genuine transformant to check for the presence of one or more genetic characters specified by the incoming plasmid DNA. The putative transformants described above were therefore assessed for high-level copper resistance and β-lactamase activity, since each phenotype (copper resistance/β-lactamase activity) is specified by genes carried on the plasmid pET13:1. The following methods were employed:

(a) High-level copper resistance. Putative pET13:1 transformants growing as patches on NEP glucose agar+0.3 mM $CuSO_4$ were subcultured by replica plating to the same medium and NEP glucose agar+1 mM $CuSO_4$. Those patched colonies which grew on the media containing both 0.3 mM and 1 mM $CuSO_4$ clearly possess high-level copper resistance. This character is presumed to be a feature of plasmid transformants carrying *CUP-1*, since copy number regulates copper resistance in yeast (Fogel, S. *et al*, 1983, *Current Genetics, 7*, p 347). It is not unreasonable to expect plasmid transformants to have a high-level of copper resistance due to the multiple copies of the plasmid genome. Those patched colonies which showed high-level copper resistance were then subjected to the β-lactamase test.

(b) The β-lactamase test for detecting β-lactamase produced by yeast strains carrying chimaeric yeast/*E. coli* plasmids is routinely applied to yeast transformants. (Chevallier, M.R. & Aigle, M., 1979, *FEBS Letters, 108*, p 179). The method described by Chevallier and Aigle (1979) is strictly adhered to and involves the following procedure:

The basis of the test is that penicillinase (β-lactamase) hydrolyses penicillin giving a reducing compound, penicilloic acid. The reducing action of penicilloic acid is rendered visible by the decoloration of a deep blue iodine-starch complex incorporated into a solid agar medium. Thus, if β-lactamase-producing strains are placed on the test medium a white halo appears around the β-lactamase-producing strain.

Test medium: Yeast nitrogen base (Difco) 0.65% w/v, glucose 0.1% w/v, soluble starch 0.2% w/v, agar 2% w/v, buffered with 0.02 M phosphate at pH 6—7.

Soft agar test medium: as above, but with 1% w/v agar.

Reagent: 3 mg/ml $I_2$; 15 mg/ml KI; 0.02 M phosphate buffer pH 7; 3 mg/ml ampicillin.

Plates containing the test medium are patched with an inoculum of putative brewers' yeast transformant. They are incubated at 30°C for 18 hours. A mixture of 4 ml melted soft agar test medium plus 1.5 ml reagent is prepared. The mixture is stirred and gently poured over the test medium. Plates, which are deep blue, are left for 1 hour at 30°C and thereafter placed at 4°C. After about 24 hours any strain producing β-lactamase shows a well defined white (colourless) halo, whereas control strains without plasmid show a very slight and limited decolouration. β-lactamase-producing transformants are therefore cleary distinguished from strains which do not possess the β-lactamase gene.

(c) Inheritable instability. A characteristic feature of yeast strains transformed with 2 µm based plasmids such as pET13:1 and pJDB207 (pJDB207 being the parental plasmid of pET13:1), is that the plasmid is inheritably unstable. The consequence of this instability is that a small proportion of yeast cells within a population segregate plasmid-free daughter cells at cell division. In the case of plasmid pET13:1, plasmid-free cells can be detected on the basis of their sensitivity to copper (NEP glucose agar+0.3 mM $CuSO_4$). Thus, copper-resistant transformants (see (a) above) are streaked on YED glucose medium and grown for 3—4 days at 27°C. Colonies arising on YED medium are then replica plated to the same medium and NEP glucose agar+0.3 mM $CuSO_4$. Colonies which have segregated the copper-resistant plasmid pET13:1 do not grow on the copper-supplemented medium. A variation of this method for evaluating the segregational phenotype of brewers' yeast transformants can be emplyed, in which putative transformants are first inoculated into NEP glucose medium (liquid medium without agar) and grown overnight at 27°C. The following day cells can be plated out on NEP glucose agar at a suitable dilution to obtain single colonies following incubation for three days at 27°C. Yeast colonies can then be replicated to NEP glucose agar and the same medium supplemented with 0.3 mM $CuSO_4$. Those brewers' yeast transformants which possess plasmid pET13:1 can be distinguished from spontaneous copper-resistant derivatives on the basis of their ability to segregate copper resistance.

Methods (a), (b) and (c) are sufficient in combination to confirm whether a putative copper-resistant transformant is genuine. It is also preferable to study the cellular morphology of all putative transformants by light microscopy. A careful comparison of transformant with the parental strain (i.e., untransformed brewers' yeast) will indicate whether the transformant is in fact a genetically modified yeast or a contaminant.

Other methods for verifying plasmid transformants could be used if desired.

The yeast transformant thus obtained identified as NCYC 240 (pET13:1) was deposited at the National Collection of Yeast Cultures, Colney Lane, Norwich, NR4 7AU, United Kingdom, on December 12th 1984 under No. NCYC1545.

A single colony of NCYC 240 (pET13:1), which was verified as a true plasmid transformant by the methods described above, was grown on NEP glucose agar with 1 MM $CuSO_4$ and inoculated into 200 ml of NEP glucose supplemented with 0.2 mM $CuSO_4$ (the liquid medium). The culture was incubated in a shake flask at 28°C for two days after which the full 200 ml was inoculated into 5 litres of the same liquid medium. Cultures were grown in stirred flasks at 20°C for four days. 5 litre cultures were then diluted, each into approximately 45 litres of lager wort. The worts were fermented for seven days and the yeast was harvested and repitched into an ale wort prepared as follows.

95% ale malt and 5% crystal malt were mashed with South Staffordshire water at 65.5°C for 90 minutes. Hops were added to 36 EBU and caramel was added to 30 EBU. The mixture was boiled for 90 minutes at 1 bar and subjected to a whirlpool stand of 30 minutes. The specific gravity of the wort at collection was 1055° at 15°C.

The yeast was pressed and pitched at 1.5 lb/barrel und the maximum fermentation temperature was 16°C. The beer was racked when the specific gravity had fallen to 1012°. The beer was conditioned at −1°C for 3 days. The beer was filtered and diluted at 1038° gravity, 1008 PG, 24 EBU bitterness and 20 EBU colour. The ethanol content was 4%. The beer was found to be acceptable to drink.

A sample of the beer was dialysed and then concentrated by freeze-drying. The freeze-dried beer was assayed for β-lactamase activity and it was found that there was no detectable β-lactamase activity.

A similar procedure was followed with NCYC 240 lacking plasmid pET13:1 (i.e. unmodified NCYC 240), with the exception that the initial yeast culture in NEP glucose did not include copper sulphate. The beers produced by fermentation using both NCYC 240 and NCYC 240 (pET13:1) were judged to be essentially similar by routine Triangular Taste Test and Flavour Profile analyses (for a review of these methods see R.J. Anderson, 1983, *Brewers Guardian, November*, p 25).

During the course of beer production with both forms of yeasts, samples of the yeast concerned were analysed in order to estimate cell number and cell viability. The results showed that there was little or no difference between the yeasts in these respects. In the case of the modified yeast, the proportion of cells containing the plasmid (pET13:1) was measured and it was found that relatively few cells lost the plasmid. Other factors were also monitored during fermentation with both the modified and the unmodified yeasts. These were: the drop in specific gravity of the wort with time, the increase in the number of cells with time and the size of the final crop of yeast. It was found that for each of those factors there was no significant difference between the use of the modified and the unmodified yeast.

Some of the modified yeast produced in the fermentation process was made available for use in a further, similar brewing process, while the excess yeast provided a source of β-lactamase.

The β-lactamase content was assessed by means of a biological assay and by means of an enzyme assay. In such assays cells are harvested by centrifugation for 10 minutes and resuspended in 0.1 M phosphate/citrate buffer pH 6.5 and disrupted using a Braun homogenizer. Glass beads and cell debris are removed by centrifugation (8000×g for 10 minutes) and the supernatant is recentrifuged (1000×g for 30 minutes). In assaying the resulting cell-free extracts by the biological assay, penicillin sensitive *E. coli* cells are plated on soft agar containing 25 μg/ml ampicillin. 25 μl of extract of NCYC 240 and NCYC 240 (pET13:1) are spotted on these plates which are subsequently incubated at 37°C. Spots of NCYC 240 (pET13:1) show strong growth of bacteria in the vicinity of the spot, spots of NCYC 240 do not. This indicates that NCYC 240 (pET13:1) cells obtained from a beer fermentation produce a substance capable of degrading penicillin and allowing the growth of sensitive *E. coli* cells, whereas cells of NCYC 240 (unmodified) do not possess this activity. The activity can be attributed to β-lactamase protein. Additional evidence that this activity can be attributed to a β-lactamase protein in NCYC 240 (pET13:1) can be obtained from the results of enzymic assays. In the first of these assays a qualitative paper disc detection system is employed, in which samples of yeast cell extracts are spotted on to Cefinase discs impregnated with the chromogenic cephalosporin, Nitrocefin, which turns from yellow to red in the presence of a β-lactamase (BBL Microbiology Systems, Becton Dickinson and Company, Oxford) (C.H. O'Callaghan *et al, Antimicrobial Agents and Chemotherapy*, 1972, *1*, p 283). Cell-free extracts of NCYC 240 (pET13:1) from a beer fermentation turn the discs from yellow to red, whereas extracts of NCYC 240 (unmodified) show no colour change on the disc, thus demonstrating the presence of β-lactamase protein in NCYC 240 (pET13:1) but not in NCYC 240. The β-lactamase activity in yeast cell extracts is quantified by using the same chromogenic cephalosporin, Nitrocefin, and the method described by C.H. O'Callaghan *et al* (1972, *Antimicrobial Agents and Chemotherapy, 1*, p 283). Enzyme reactions are performed at 37°C in a 1 cm cell containing a total volume of 1 ml Nitrocefin solution (51.6 μg of Nitrocefin 87/312 per ml in 0.05 M phosphate buffer, pH 7) to which 20 μl of cell-free yeast extract is added. The change in optical density of the reaction mixture is determined at 386 nm and 482 nm using a Beckman DU 7 spectrophotometer. In this way crude cell-free extracts of NCYC 240 (pET13:1) from a beer fermentation are capable of destroying 4.87 n moles of Nitrocefin 87/312/min/mg protein at 37°C and pH 7.0 (protein estimates are obtained from the absorption of ultra violet light at 230 and 260 nm according to V.F. Kalb and R.W. Bernlohr (1977, *Analytical Biochemistry, 82*, p 362). Crude cell extracts of NCYC 240 (unmodified) and boiled extracts of NCYC 240 (pET13:1) (unmodified) and boiled extracts of NCYC 240 (pET13:1) (20 mins at 100°C do not possess any β-lactamase activity.

Procedures similar to those described above in detail in relation to NCYC 240 have also been carried out with a proprietary strain of brewers' yeast, and the results obtained were very similar.

There now follows a description of the modification of NCYC 240 to enable it to produce a different protein material, namely a β-glucanase. An endo-1,3-1,4-β-D-glucanase (EC 3.2.1.73) is an enzyme which catalyses the hydrolysis of alternating sequences of ß-1,3 and ß-1,4-linked -ß-D-glucan, as in barley β-glucan and lichenan. The unique action of this enzyme precludes its ability to hydrolyse repeating sequences of β-1,3-linked glucan, as in laminarin, and β-1,4-linked glucan, as in carboxymethylcellulose (Barras, D.R., 1969, In "Cellulases and Their Applications", *156th meeting of the American Chemical Society*, Sept. 11—12, 1968, Atlantic City, p 105).

The Gram-positive bacterium *Bacillus subtilis* produces an extra-cellular endo-1,3-1,4-β-D-glucanase which behaves in a similar fashion to that described above (Moscatelli, E.A. *et al*, 1961, *Journal of Biologial Chemistry, 236*, p 2858; Rickes, E.L. *et al*, 1962, *Archives of Biochemistry and Biophysics, 69*, p 371).

A chromosomal *B. subtilis* B-glucanase gene has been isolated by gene cloning from a strain of

*B. subtilis* entitled NCIB 8565 (Hinchliffe, E., 1984, *Journal of General Microbiology, 130,* p 1285). The active gene was found to reside upon a 3.5 kilo-base (kb) pair restriction-endonuclease-*Eco* RI-fragment of DNA, which expressed a functional enzyme in *E. coli.* The cloned β-glucanase gene was shown to encode an enzyme specific for the hydrolysis of barley β-glucan, and was found to be predominantly extracytoplasmic in location in *E. coli* (Hinchliffe, 1984).

More recently the cloned β-glucanase gene has been located by deletion analysis on a 1.4 kb restriction endonuclease *Pvu*l-*Cla*l DNA fragment. A similar location has been assigned to a *B. subtilis* β-glucanase gene isolated from strain NCIB 2117 (Cantwell, B.A. & McConnell, D.J., 1983, *Gene, 23,* p 211). A more precise molecular characterization by DNA sequence analysis of the NCIB 2117 has recently been reported (Murphy, N. *et al*, 1984, *Nucleic Acids Research, 12,* p 5355).

Yeasts, including *S. cerevisiae,* produce several different types of β-glucanase; however, none is able to hydrolyse β-1,3-1,4 - linked glucan (Abd-E1-A1, A.T.H. & Phaff, H.J., 1968, *Biochemical Journal, 109,* p 347). It must therefore follow that yeast does not produce an endo-1,3-1,4-β-D-glucanase. The cloned β-glucanase gene of *B. subtilis* has therefore been introduced into *S. cerevisae,* and it has been demonstrated that the gene is capable of encoding a biologically active protein in *S. cerevisiae* and that the enzyme activity is characteristic of that found in *B. subtilis* and *E. coli* (Hinchliffe, E. & Box, W.G., 1984, *Current Genetics, 8,* p 471). The expression of the cloned β-glucanase gene in *S. cerevisiae* is inefficient, relative to the amounts of biologically active enzyme produced in both *B. subtilis* and *E. coli* harbouring the cloned β-glucanase gene. However, the enzymic activity in yeast can only be detected in crude cell extracts of yeast harbouring the cloned gene (Hinchliffe, E. & Box, W.G., 1984). This may mean that the enzyme produced by yeast is incapable of being secreted from the cell and is intra-cellular in nature; unlike the enzyme produced by bacteria, which is extra-cellular.

To introduce the β-glucanase gene of *B. subtilis* into brewers' yeast NCYC 240, use was made of the shuttle vector pET13:1, that can replicate in both *E. coli* and *S. cerevisiae,* as mentioned above. The 3.5 kb *Eco* RI DNA fragment present in plasmid pEHB3 was subcloned by *in vitro* rearrangement into the single Bam HI site of pET13:1, as illustrated in more detail in the accompanying drawings. In the gene maps in the drawing the radially hatched arcs represent DNA from *B. subtilis* that carries the β-glucanase gene (βG), the broad, unfilled arcs represent chromosomal DNA indicating the location of *LEU-2* and *CUP-1* genes, (Henderson, R.C.A., 1983, "The Genetics and Applications of Copper Resistance in Yeast", Ph.D thesis, University of Oxford), and the narrow arcuate black lines represent 2 μm plasmid DNA and the thick arcuate black lines represent *E. coli* vector DNA sequences. Treatment with T4 DNA ligase following *Eco* RI digestion of pEHB3 (Hinchliffe, E., 1984, *Journal of General Microbiology, 130,* p 1285) was performed under dilute DNA concentrations, thus favouring circularization of the two products of *Eco* RI digestion. One of those products is a circle of the DNA from the broad black arc of pEHB3. On digestion of the products with the restriction endonuclease *Bg*/II that circle was broken at the *Bg*/II site to form a 3.5 kb linear fragment. Meanwhile pET13:1 was digested and the resulting linear fragment was ligated with the linear fragment from pEHB3, using T4 DNA ligase. That digestion and ligation were carried out at higher DNA concentrations, which favour recombination of the rearranged *B. subtilis* DNA in the Bam HI site of pET13:1 (Henderson, R.C.A., 1983, "The Genetics and Applications of Copper Resistance in Yeast", Ph.D. thesis, University of Oxford). Ligation occurs because the endonucleases Bam HI and *Bg*/II generate mutually compatible cohesive ends which join to form Bam HI/*Bg*/II hybrid sites which are not recognized by either Bam HI or *Bg*/II. Transformants were selected in *E. coli* strain HB101 as being ampicillin-resistant, tetracycline-sensitive and β-glucanase positive in *E. coli,* thus enabling them to be distinguished from pEHB3 in *E. coli.* The orientation of insertion of the rearranged *Eco* RI fragment in pEHB10 was determined by restriction endonuclease digestion followed by agarose gel electrophoresis. The new plasmid has been designated pEHB10.

Plasmid DNA was isolated from HB101 harbouring the hybrid plasmid pEHB10; this DNA was transformed into the brewers' yeast NCYC 240 as described previously. Resistance to copper was selected, as also described above. Plasmid transformants of NCYC 240 were verified by a combination of high-level resistance determinations and β-lactamase assays, thus NCYC 240 (pEHB10) was derived.

Single colonies of NCYC 240 (pEHB10), NCYC 240 (pET13:1) and NCYC 240 were inoculated into 200 ml of NEP glucose (supplemented with 0.2 mM $CuSO_4$ where appropriate). Cultures were incubated while being shaken at 27°C for 2 days, after which time they were inoculated each into the 2 litres of the same medium. After 3 days' growth at 27°C cells were harvested by centrifugation and washed twice in 0.1 M phosphate/citrate buffer at pH 6.4 prior to cell disruption in a Braun homogenizer. Supernatants were prepared as described previously with the exception that each was dialysed overnight against 2×21 of 0.1 M phosphate/citrate: pH 6.4. Crude cell extracts of the three NCYC 240 yeast were then subjected to β-glucanase assays as described by Hinchliffe & Box (1984). These assays demonstrated β-glucanase activity associated with cell-free extracts of NCYC 240 (pEHB10) (1.17 n moles of reducing sugar liberated from barley B-glucan/min/mg protein at 40°C and pH 6.2), but no activity in cell-free extracts of either NCYC 240 (pET13:1) or NCYC 240.

The yeast transformant thus obtained identified as NCYC 240 (pEHB10) has been deposited at the National Collection of Yeast Cultures, Colney Lane, Norwich NR47AU, United Kingdom on December 12th 1984 under No. 1546.

A sample of the NCYC 240 (pEHB10) yeast was grown in the manner described above and used in a brewing process similar to that described above in relation to NCYC 240 (pET13:1). The process yielded beer that was acceptable to drink and that contained substantially no endo-1,3-1,4-β-D-glucanase. Yeast from the brewing process was shown to contain the plasmid pEHB10, specifying the production of β-glucanase. (1 n mole reducing sugar liberated from barley β-glucan/min/mg protein at 40°C and pH 6.4), so that part of it could be re-cycled (that is used in a subsaquent brewing operation) and part of it could be used as a source of the enzyme. Furthermore, crude cell extracts of NCYC 240 (pEHB10) derived from the brewing process contain β-lactamase enzyme activity (2.33 n moles of Nitrocefin 87/312 destroyed/min/mg protein at 37°C and pH 7.0) as well as β-glucanase enzyme activity. This demonstrates the feasibility of producing more than one heterologous protein at the same time in a genetically modified brewing yeast, such as NCYC 240.

Endo-1,3-1,4-β-D-glucanase obtained from *B. subtilis* is currently marketed as an enzyme preparation for use in the brewing industry in alleviating problems associated with the presence of unwanted β-glucan. The process described above may therefore be used to produce this enzyme for the same purpose.

## Claims

1. Process for the production of ethanol and a protein or peptide which is heterologous to yeast which comprises fermenting an aqueous sugar-containing medium with an industrial *Saccharomyces cerevisiae* or *S. carlbergensis* yeast strain which has been genetically modified to be capable of expressing a heterologous protein or peptide, recovering the ethanol so formed, and obtaining the said heterologous protein or peptide from the fermentation products.

2. Process according to claim 1 in which the ethanol is recovered in the form of an aqueous potable liquid which is substantially free from yeast and from the said heterologous protein or peptide and which contains substantially all the water and ethanol of the said fermented medium.

3. Process according to claim 1 in which the ethanol is recovered from the said fermented medium in the form of an ethanolic distillate.

4. Process according to claim 2 in which the aqueous sugar-containing medium contains maltose as the major sugar present.

5. Process according to claim 4 in which the aqueous sugar-containing medium is a barley malt-based beer wort.

6. Process according to claim 2, 4 or 5 in which the fermentation is effected at 8 to 25°C.

7. Process according to claim 3 in which the aqueous sugar-containing medium is a fermentation medium for the production of potable distilled ethanol or power ethanol.

8. Process according to claim 7 in which the said medium is based on grain, potatoes, cassava, sugar cane, or sugar beet, optionally pretreated to convert cellulose and/or starch therein into fermentable sugars.

9. Process according to any one of claims 1 to 8 in which the fermentaion is a substantially anaerobic fermentation.

10. Process according to any of claims 1 to 9 in which the said heterologous protein or peptide is obtained as protein or peptide retained in the yeast produced during the fermentation.

## Patentansprüche

1. Verfahren zur Herstellung von Ethanol und eines zu Hefe heterologen Proteins oder Peptids durch Fermentieren eines wäßrigen, zuckerhaltigen Mediums mit einem Industrie-Saccharomyces cerevisiae- oder S. carlsbergensis-hefestamm, der derart genetisch modifiziert wurde, daß er zur Expression eines heterologen Proteins oder Peptids fähig ist, Gewinnen des gebildeten Ethanols und Gewinnen des heterologen Proteins oder Peptids aus den Fermentationsprodukten.

2. Verfahren nach Anspruch 1, dadurch gekennzeichnet, daß das Ethanol in Form einer praktisch hefefreien und praktisch das gesamte Wasser und das gesamte Ethanol des fermentierten Mediums enthaltenden wäßrigen trinkbaren Flüssigkeit gewonnen wird.

3. Verfahren nach Anspruch 1, dadurch gekennzeichnet, daß das Ethanol aus dem fermentierten Medium in Form eines Ethanoldestillats gewonnen wird.

4. Verfahren nach Anspruch 2, dadurch gekennzeichnet, daß das wäßrige, zuckerhaltige Medium Maltose als hauptsächlich vorhandenen Zucker enthält.

5. Verfahren nach Anspruch 4, dadurch gekennzeichnet, daß es sich bei dem wäßrigen, zuckerhaltigen Medium un eine Bierwürze auf Gerstenmalzbasis handelt.

6. Verfahren nach Anspruch 2, 4 oder 5, dadurch gekennzeichnet, daß die Fermentierung bei 8° bis 25°C durchgeführt wird.

7. Verfahren nach Anspruch 3, dadurch gekennzeichnet, daß es sich bei dem wäßrigen, zuckerhaltigen Medium um ein Fermentationsmedium für die Gewinnung von destilliertem Trinkethanol oder Antriebsethanol handelt.

8. Verfahren nach Anspruch 7, dadurch gekennzeichnet, daß das Medium auf Korn, Kartoffeln, Maniok, Zuckerrohr oder Zuckerrüben, die gegebenenfalls zur Umwandlung von darin enthaltener Cellulose und/oder Stärke zu vergärbaren Zuckern vorbehandelt wurden, basiert.

9. Verfahren nach einem der Ansprüche 1 bis 8, dadurch gekennzeichnet, daß die Fermentation im wesentlichen anaerob geführt wird.

10. Verfahren nach einem der Ansprüche 1 bis 9, dadurch gekennzeichnet, daß das heterologe Protein oder Peptid als in der während der Fermentierung gebildeten Hefe enthaltenes Protein oder Peptid gewonnen wird.

**Revendications**

1. Procédé pour la production d'éthanol et d'une protéine ou d'un peptide qui sont hétérologues à une levure, caractérisé en ce qu'il comprend la fermentation d'un milieu aqueux contenant des sucres avec une souche de levure industrielle, à savoir *Saccharomyces cerevisiae* ou *S. carlsbergensis*, qui a été modifiée génétiquement pour être capable d'exprimer une protéine ou un peptide hétérologues, la récupération de l'éthanol ainsi formé et l'obtention de cette protéine ou de ce peptide hétérologues à partir des produits de fermentation.

2. Procédé suivant la revendication 1, caractérisé en ce que l'éthanol est récupéré sous la forme d'un liquide aqueux potable, qui est pratiquement dépourvu de levure et de cette proteine ou de ce peptide hétérologues et qui contient pratiquement la totalité de l'eau et de l'éthanol de ce milieu fermenté.

3. Procédé suivant la revendication 1, caractérisé en ce que l'éthanol est récupéré à partir de ce milieu fermenté sous la forme d'un distillat éthanolique.

4. Procédé suivant la revendication 2, caractérisé en ce que le milieu aqueux contenant des sucres contient du maltose en tant que principal sucre présent.

5. Procédé suivant la revendication 4, caractérisé en ce que le milieu aqueux contenant des sucres est un moût de bière à base de malt d'orge.

6. Procédé suivant les revendications 2, 4 ou 5, caractérisé en ce que la fermentation est effectuée de 8 à 25°C.

7. Procédé suivant la revendication 3, caractérisé en ce que le milieu aqueux contenant des sucres est un milieu de fermentation pour la production d'éthanol distillé potable ou d'éthanol carburant.

8. Procédé suivant la revendiction 7, caractérisé en ce que ce milieu est à base de grain, pommes de terre, manioc, sucre de canne ou sucre de betterave, éventuellement prétraités pour convertir la cellulose et/ou l'amidon contenus dans ceux-ci en sucres fermentescibles.

9. Procédé suivant l'une quelconque des revendications 1 à 8, caractérisé en ce que la fermentation est une fermentation pratiquement anaérobie.

10. Procédé suivant l'une quelconque des revendications 1 à 9, caractérisé en ce que cette protéine ou ce peptide hétérologues sont obtenus en tant que protéine ou peptide retenus dans la levure produite pendant la fermentation.

CONSTRUCTION OF THE β-GLUCANASE CUP-1 PLASMID pEHB10

**pEHB 3** 9·5 kbp

E C B A Pv P C E Pv Bg A P Tc Ap βG

**pET 13:1** 11·6 kbp

Ap P E E K LEU 2 Pv B X H E H S S X CUP1 CUP1 K S S X K S

1 Eco RI
2 LIGASE

1 Bam HI

1 Bgl II
2 LIGASE

**pEHB10** 15·1 kbp

Pv Ap P Pv E C E P E βG/ LEU 2 E K CUP1 CUP1 S X K S S X K S X H E S H

Bacillus subtilis DNA (▥▥▥),
pAT153/pBR325 DNA (▮▮▮),
yeast chromosomal DNA (▭),
yeast 2 μm plasmid DNA (——).
The following gene locations
are indicated by arrows:
β-Glucanase (βG), β-lactamase
(Ap) tetracycline resistance
gene (Tc).
Restriction endonuclease cut
sites are shown as follows:
AvaI (A), BamHI (B), BglII (Bg),
ClaI (C), EcoRI (E), HindIII (H),
KpnI (K), PvuI (P), PvuII (Pv),
Sau3A (S) and XbaI (X).